# EUROPÄISCHE PATENTSCHRIFT

(11) **EP 0 845 238 B1**
(45) Veröffentlichungstag und Bekanntmachung des Hinweises auf die Patenterteilung: **29.08.2001**
(21) Anmeldenummer: 97119015.2
(22) Anmeldetag: 31.10.1997
(51) Int. Cl.: A61B 5/00, A61B 18/00, A61N 5/06, A61B 1/00

(54) **Vorrichtung zur endoskopischen Diagnose und Behandlung von Gewebe**
Device for endoscopic diagnostics and for treatment of tissue
Dispositif destiné au diagnostic par endoscopie et au traitement de tissus

(30) Priorität: 08.11.1996 DE 19646236
(43) Veröffentlichungstag der Anmeldung: 03.06.1998
(73) Patentinhaber: Richard Wolf GmbH, 75438 Knittlingen (DE)
(72) Erfinder: Dütting, Kaspar, 70180 Stuttgart (DE); Schurr, Marc, 72074 Tübingen (DE); Buess, Gerhard, 72074 Tübingen-Bebenhausen (DE); Müller, Gerhard, 14129 Berlin (DE); Wagner, Bernd, 25582 Looft (DE)
(74) Vertreter: Vollmann, Heiko, Dipl.-Ing.

(56) Entgegenhaltungen:
- DE-A- 3 331 586
- DE-A- 3 740 318
- DE-A- 4 102 614
- DE-A- 4 321 786
- US-A- 5 309 895
- US-A- 5 413 108

## Beschreibung

Die Erfindung betrifft eine Vorrichtung zur endoskopischen Diagnose und Behandlung von Gewebe gemäß den im Oberbegriff des Anspruchs 1 angegebenen Merkmalen.

Endoskopische Vorrichtungen der eingangs erwähnten Art - cine solche Vorrichtung ist beispielsweise aus US-PS 5,413,108 bekannt - werden heutzutage beispielsweise in der Rektoskopie angewendet. Dabei wird ein schlanker distaler Endabschnitt eines in der Regel flexiblen Endoskopes über eine Körperöffnung in den Körper ein- und bis zur zu diagnostizierenden bzw. zu behandelnden Stelle hingeführt. Der zu diagnostizierende Gewebebereich, beispielsweise die Darmwand, wird mit mindestens zwei Diagnoselasern unterschiedlicher Wellenlänge bestrahlt, wonach das remittierte Licht - sei es durch Reflexion oder auch durch Fluoreszenz - wellenlängenabhängig erfaßt wird. Die Empfangssignale werden bildpunktabhängig durch Subtraktion oder Quotientenbildung miteinander verknüpft, wodurch die Topologie des zu diagnostizierenden Gewebeabschnitts ausgeschaltet wird. Es werden also durch diese elektronischen Signalverknüpfungen Intensitätsunterschiede des empfangenen Bildes, wie sie beispielsweise durch Schatten, Erhöhungen, Vertiefungen und dergleichen entstehen, ausgeschaltet. Das verbleibende Bild bzw. elektrische Signal ermöglicht aufgrund der wellenlängenabhängigen Remissionserscheinungen von gesundem und malignem Gewebe die erwünschte Diagnose. Nach erfolgter Diagnose kann das maligne Gewebe direkt durch einen Behandlungslaser verdampft, also entfernt werden. Mittels der Diagnoselaser kann unmittelbar danach eine Kontrolle erfolgen, ob das maligne Gewebe vollständig entfernt worden ist oder nicht.

Der vorrichtungsmäßige Aufbau dieser aus der US-PS 5,413,108 bekannten Anordnung ist so, daß sowohl die Diagnose- als auch die Behandlungslaser sowie die Empfangseinrichtungen außerhalb des Endoskopes liegen und über Lichtleiter mit dem distalen Endoskopende verbunden sind. Die Wellenlängenseparation erfolgt über Filter, die in einer in den Strahlengang eingreifenden Scheibe liegen, welche motorisch antreibbar ist.

Eine ähnliche Anordnung ist aus der US-PS 5,309,895 bekannt. Dort ist ein CCD im distalen Endoskopende angeordnet, um eine möglichst hohe Lichtausbeute zu erzielen, doch ist das bei dieser Vorrichtung empfangene Lichtsignal noch schwächer, da die Beleuchtung nur periodisch erfolgt.

Eine ähnliche Anordung ist aus GB 2125986 A bekannt. Bei dieser Vorrichtung ist der Behandlungslaser nicht durch einen gesonderten Arbeitskanal hindurchzuführen, sondern bereits intergriet.

Nachteilig bei den vorerwähnten Vorrichtungen ist insbesondere der komplizierte und technisch aufwendige Aufbau. Das durch Reflexion oder Fluoreszenz emittierte Licht ist äußerst schwach und muß daher in technisch aufwendiger Weise verstärkt werden, bevor es empfangen und ausgewertet werden kann. Dies führt letztlich dazu, daß die Diagnoseeinrichtung eine vergleichsweise geringe Empfindlichkeit aufweist oder aber nicht mit der notwendigen Zuverlässigkeit arbeitet. Darüber hinaus ergeben sich bei den vorbekannten Vorrichtungen sowohl bei der Diagnose als auch bei der Therapie häufig Probleme hinsichtlich der Genauigkeit des Diagnose- bzw. Therapieortes.

Ausgehend von diesem Stand der Technik liegt der Erfindung die Aufgabe zugrunde, eine gattungsgemäße Vorrichtung so auszubilden, daß einerseits eine hohe Genauigkeit bzw. Empfindlichkeit, jedoch andererseits ein kostengünstiger und robuster Aufbau erreicht wird.

Diese Aufgabe wird gemäß der Erfindung durch die im kennzeichnenden Teil des Anspruches 1 angegebenen Merkmale gelöst.

Demgemäß sieht die erfindunggemäße Lösung mindestens eine Scaneinrichtung vor, welche die Lichtstrahlen des Diagnoselasers über ein Diagnosefeld lenkt, und eine Fotodiode als Empfangseinrichtung. Es wird also nicht gleichzeitig das gesamte Diagnosefeld bestrahlt, sondern es wird der Laserstrahl mittels der Scaneinrichtung nach Art der Flying-Spot-Verfahren über das Diagnosefeld geführt. Hierdurch entsteht punktuell auf dem Diagnosefeld eine im Vergleich zum Stand der Technik beim Einsatz eines vergleichbaren Diagnoselasers eine wesentliche höhere Beleuchtungsstärke. Dies bedingt widerrum eine stärke Remission, weshalb das von der Empfangseinrichtung aufgenommene Signal stärker und damit weniger aufwendig in der Aufnahme und Weiterverarbeitung ist.

Dieser Effekt wird noch dadurch verstärkt, dass als Empfangseinrichtung für den jeweiligen Diagnoselaser nicht ein CCD, das gleichzeitig die Remission des gesamten Diagnosefeldes erfasst, verwendet wird, sondern eine Fotodiode. Diese Fotodiode, die im Vergleich zu einem CCD einen wesentlichen größeren Aperturwinkel und damit eine wesentlich höhere Empfindlichkeit hat, empfängt zeitlich gesehen nur das remittierte Licht bzw. die Fluoreszenz des Punktes des Diagnosefeldes, der mittels der Scaneinrichtung gerade bestrahlt wird. Es ergibt sich also auch empfangseinrichtungsseitig ein wesentlich stärkeres Signal, da das gesamte remittierte Licht von nur einer oder beispielsweise drei- wenn drei Dreidiagnoselaser mit jeweils einer eigenen Empfangseinrichtung vorgesehen werden- Fotodioden aufgenommen wird, was zu einem wesentlich stärkeren und rauschärmeren Signal führt, als wenn vergleichsweise ein CCD eingesetzt wird, bei dem das remittierte Licht auf eine Vielzahl von Fotoelementen verteilt wird.

Die bildweise Auswertung erfolgt bei der erfindungsgemäßen Vorrichtung mittels der Steuer- und Auswerteinrichtung, und zwar in Abhängigkeit der Zeit, da jedem Empfangssignal zeitabhängig ein bestimmter Bildpunkt in einer Bildpunktmatrix zugeordnet werden kann, entsprechend der jeweiligen Stellung der Scaneinrichtung. Ferner ermöglicht diese Anordung eine Integration einer Autofocuseinrichtung ohne zusätzliche Fotodioden einzusetzen.

Auch sieht die Erfindung vor, die Empfangseinrichtung bzw. beim Einsatz mehrerer Empfangseinrichtungen diese im distalen Endabschnitt des endoskopischen Teils der Vorrichtung anzuordnen. Hierdurch kann das durch Fluoreszenz oder Reflexion emittierte Licht auf kürzestem Weg und unter Vermeidung aufwendiger optischer Vorrichtungen den Empfangsvorrichtungen zugeführt werden, so daß auf die sonst üblichen aufwendigen Verstärker verzichtet werden kann. Die Lichtführung innerhalb des distalen Endabschnittes vom Lichteintritt bis zur Empfangseinrichtung kann in der Regel ausschließlich über Spiegel erfolgen, so daß ein Großteil dieses Lichtes der Empfangseinrichtung bzw. den Empfangseinrichtungen zugeführt werden kann. Dabei ist insbesondere wesentlich, daß auf den Einsatz von den sonst üblichen Lichtleitern im Bereich zwischen Lichteintritt und Empfangseinrichtung vollständig verzichtet werden kann, wodurch der sonst übliche kleine Akzeptanzwinkel wesentlich vergrößert und damit die Einkoppelverluste ganz erheblich verringert werden können. Die erfindungsgemäße Vorrichtung weist damit schon konstruktionsbedingt eine wesentlich höhere Empfindlichkeit auf als die aus dem Stand der Technik bekannten, wobei der Aufbau schon dadurch erheblich kostengünstiger und einfacher ist, daß auf die sonst üblichen Lichtverstärker vollständig verzichtet werden kann.

Die Genauigkeit hinsichtlich des Diagnose- und Therapieortes kann gemäß der Erfindung weiterhin dadurch erhöht werden, daß die Strahlengänge des ausgesandten und einfallenden Lichtes am distalen Ende der Vorrichtung, d. h. die Strahlengänge auch von Diagnose- und Behandlungslaser in Übereinstimmung gebracht werden. Das Zusammenführen der Strahlengänge hat darüber hinaus auch konstruktive Vorteile und ermöglicht eine schlanke Ausgestaltung der Vorrichtung insbesondere im distalen Endbereich.

Grundsätzlich kann die erfindungsgemäße Vorrichtung mit einem Diagnoselaser und einer Empfangseinrichtung betrieben werden, wenn sowohl der Diagnoselaser als auch die Empfangseinrichtung so getaktet eingesetzt werden, daß für ein bestimmtes Zeitintervall Licht einer ersten Wellenlänge erzeugt und empfangen und für ein weiteres Zeitintervall Licht einer zweiten Wellenlänge erzeugt und empfangen wird, so wie es beispielsweise in US-PS 5,413,108 beschrieben ist. Dies kann in extremen Fällen jedoch zu Bewegungsunschärfen führen, weshalb gemäß der Erfindung bevorzugt vorgeschlagen wird, mindestens zwei Diagnoselaser unterschiedlicher Wellenlänge vorzusehen und jedem Diagnoselaser eine im distalen Endabschnitt der Vorrichtung angeordnete Empfangseinrichtung zuzuordnen. Dann können beide Diagnoselaser gleichzeitig betrieben werden, entsprechend erfolgt der Empfang, so daß bei der elektronischen Signalverknüpfung zur Topologieeliminierung stets ein nahezu Echtzeitbild entsteht.

Für viele Anwendungen ist die Auswertung mittels stark unterschiedlicher Absorption/Remission der zwei Diagnosewellenlängenbereiche vorteilhaft. Hier genügt zur Ausschaltung der Topologie des bestrahlten Gewebebereichs grundsätzlich die Bestrahlung mit zwei unterschiedlichen Wellenlängen und eine entsprechende Auswertung. In manchen Anwendungen ist es jedoch auch vorteilhaft, wenn drei Diagnoselaser unterschiedlicher Wellenlängen vorgesehen sind, die vorzugsweise die Wellenlängenbereiche rot, grün und blau abdecken. Dann kann nämlich mit den vom remittierten Licht erhaltenen Empfangssignalen in diesen drei Wellenlängenbereichen zusätzlich ein natürliches Farbbild beispielsweise auf einem Monitor dargestellt werden. Der behandelnde Arzt kann somit neben dem rechnerisch durch Subtraktion, Quotientenbildung oder anderer geeigneter Weise gebildeten Diagnosebild auch seine aus dem natürlichen Betrachtungsbild gesammelten Erfahrungen einbringen.

Um ein exaktes, hochauflösendes Diagnosebild zu erhalten, werden die Diagnoselaserstrahlen nach Art des Flying-Spot-Verfahrens über ein Diagnosefeld geleitet, in dessen Mittelpunkt der Strahlengang des Therapielasers mündet. Um dies zu erreichen weist die Vorrichtung vorteilhaft innerhalb des distalen Endabschnittes Scaneinrichtungen auf, welche die Laserstrahlen der Diagnoselaser vor dem Auftreffen horizontal sowie vertikal ablenken. Die jeweilige Ablenkung wird elektronisch erfaßt, so daß dem Diagnosefeld zeitabhängig bestimmte Diagnosepunkte zugeordnet werden können. Entsprechend werden die Signale der Empfangseinrichtungen verknüpft, so daß eine bildgebende Punktmatrix entsteht, die durch frequente Abfrage, beispielsweise dreimal pro Sekunde, als Bild einem Monitor zuführbar ist, der ungeachtet der Abfragefrequenz beispielsweise mit 50 oder 100 Hz zum Erhalt eines ruhigen Bildes getaktet werden kann. Derartige Mikroscanner sind an sich bekannt. Es wird in diesem Zusammenhang auf "Mikrosystemtechnik 1994 bis 1999, Programm im Rahmen des Zukunftkonzeptes Informationstechnik", herausgegeben vom Bundesministerium für Forschung und Technologie - Öffentlichkeitsarbeit aus Januar 1994 (ISBN 3-88135-276-7), verwiesen, dort insbesondere auf Seite 81. Es handelt sich hierbei um Halbleiterspiegel, die nach Art einer Wippe angeordnet sind und an der Unterseite Elektroden aufweisen, so daß durch elektromagnetische Beaufschlagung eine Wippbewegung zur einen bzw. zur anderen Richtung erfolgt. Diese Spiegel werden üblicherweise im Resonanzbereich betrieben, so daß die Auswertelektronik lediglich an die Resonanzfrequenz anzupassen ist. Darüber hinaus ist es auch möglich, diese Spiegel durch gezielte elektromagnetische Beaufschlagung von der einen oder anderen Seite gezielt zu bewegen, um den gewünschten Scaneffekt zu erreichen, d. h. den Auftreffpunkt eines Diagnoselaserstrahls im Diagnosefeld gezielt zu beeinflussen.

Für eine ortsgenaue Diagnose ist es aber darüber hinaus von Vorteil, wenn jedem Diagnoselaser eine Empfangseinrichtung zugeordnet ist, die selektiv nur für den Empfang des Lichtes der Wellenlänge vorgesehen ist, die von diesem Laser ausgestrahlt wird. Eine besonders günstige Anordnung zur Erzielung eines intensiven und gut detektierbaren Signals ergibt sich dann, wenn die Empfangseinrichtungen innerhalb des distalen Endabschnittes angeordnet sind, und zwar zwischen dem distalen Ende der Vorrichtung und den Scaneinrichtungen. Der Empfang erfolgt mit üblichen (vergleichsweise großflächigen) Fotodioden, so dass als Empfangssignal stets das gesamte vom Gewebe remittierte Licht des jeweiligen Diagnoselasers zur Verfügung steht. Die Auswertung als Bildpunktmatrix erfolgt entsprechend der zeitlichen Zuordnung der einzelnen Bildpunkte.

Konstruktiv besonders günstig ist es, wenn jeder Empfangseinrichtung eine Fotodiode zugeordnet wird, wobei die wellenlängenabhängige Aufteilung des remittierten Lichtes in an sich bekannter Weise über halbdurchlässige, dikroitisch beschichtete Prismen bzw. Prismenanordnungen erfolgen kann. Vorteilhaft ist die Auswertung der Empfangssignale mit der Freigabe oder Auslösesteuerung des Behandlungslasers verknüpft, so daß beispielsweise eine Auslösung des Behandlungslasers nur dann möglich ist, wenn ein vorgegebener (malignes Gewebe kennzeichnender) Schwellwert innerhalb des den Behandlungslaser umfassenden Bildbereiches überschritten wird. Andererseits kann auch ein aktiver Eingriff in die Steuerung des Behandlungslasers dergestalt erfolgen, daß eine automatische Auslösung beim Überschreiten eines vorgegebenen Schwellwerts erfolgt.

Für den praktischen Einsatz der Vorrichtung besonders zweckmäßig ist das Vorsehen einer an sich bekannten Autofokuseinrichtung. Diese Autofokuseinrichtung kann unter weitgehender Ausnutzung vorhandener Bauteile in den distalen Endabschnitt integriert werden, wenn auch deren Strahlengang mit dem der Diagnoselaser sowie dem des Behandlungslasers im distalen Ende der Vorrichtung zusammenfällt. Bevorzugt ist die Fokussierungslinse der Autofokuseinrichtung im distalen Endabschnitt direkt vor dem distalen Abschlußfenster angeordnet und durch beispielsweise eine übliche Magnet-Spulenanordnung durch elektromagnetische Beaufschlagung in Achsrichtung des Strahlengangs zum Zwecke der Fokussierung verlagerbar.

Bevorzugt arbeitet die Autofokuseinrichtung mit dem am intensivsten remittierten Wellenlängenbereich (geringste Absorption z. B. 1,3 µm im Infrarotbereich und 0,35 µm im UV-Bereich), wobei hierzu das vom entsprechenden Diagnoselaser ausgesandte Licht benutzt werden kann, wenn eine entsprechende zeitliche Taktung zwischen dem für die Autofokuseinrichtung erforderlichen Empfang und dem für die Diagnose erforderlichen Empfang erfolgt. In diesem Fall können die Photodioden der Empfangseinrichtungen auch Teil der Autofokuseinrichtung bilden. Dies führt nicht nur zu einer kostengünstigen und platzsparenden Ausbildung des endoskopischen Endabschnitts, sondern auch zu einer besonders hohen Genauigkeit der Autofokuseinrichtung und somit einer hohen Zielgenauigkeit der gesamten Vorrichtung.

Die Erfindung ist nachfolgend anhand von in der Zeichnung dargestellten Ausführungsbeispielen näher erläutert. Es zeigen:
- Fig. 1: ein vereinfachtes Blockschaltbild der erfindungsgemäßen Vorrichtung,
- Fig. 2: in stark vereinfachter schematischer Darstellung die erfindungsgemäße Vorrichtung mit im Längsschnitt dargestelltem distalen Endabschnitt,
- Fig. 3: eine andere Ausführung des distalen Endabschnitts im Längsschnitt und
- Fig. 4: eine weitere Ausführungsvariante in Darstellung nach Figur 3.

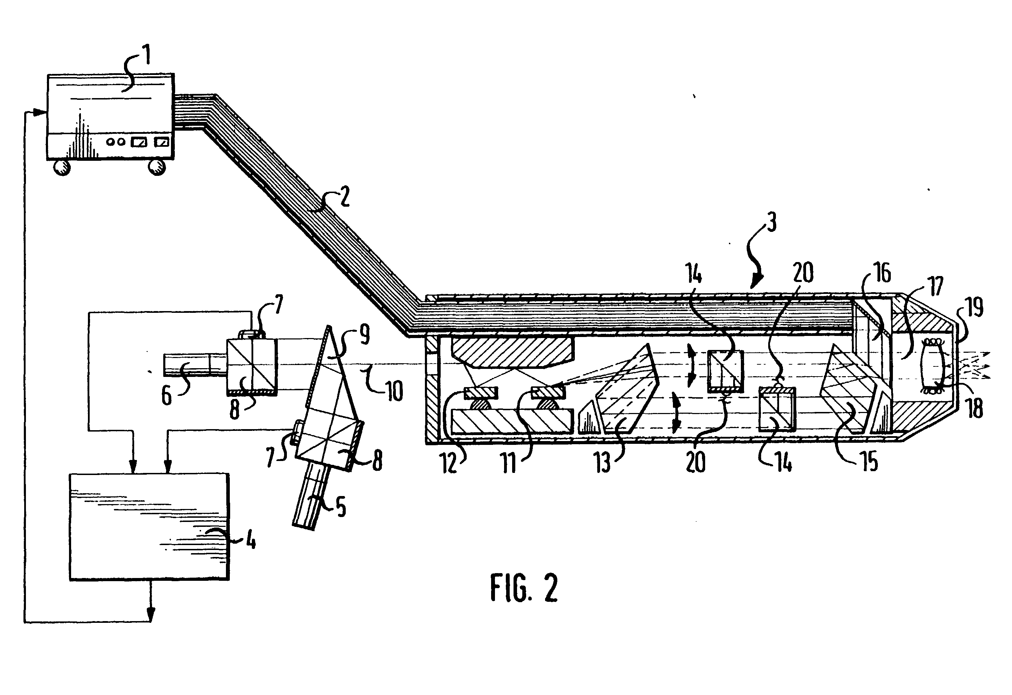

Die Vorrichtung gemäß Figur 2 weist einen Behandlungslaser 1 auf. Es handelt sich hierbei um einen im 1,06 *µ*m-Bereich arbeitenden Nd-YAG-Laser. Das Licht des Behandlungslasers 1 wird über ein Multimode-Faserbündel 2 geleitet, das in einem distalen Endabschnitt 3 eines hier nicht im einzelnen dargestellten und beschriebenen endoskopischen Instrumentes, beispielsweise eines flexiblen Endoskopes ähnlich dem aus US-PS 5,413,108 bekannten, mündet. Außerhalb des endoskopischen Instrumentes sind weiterhin eine elektronische Auswert- und Steuereinheit 4 sowie zwei Diagnoselaser 5 und 6 angeordnet. Jeder Diagnoselaser ist über einen aus zwei Prismen aufgebauten Strahlteilerblock 8 mit einer Empfangseinrichtung für remittiertes Licht in Form einer Photodiode 7 verbunden. Die Diagnoselaser 5 und 6 mit ihren zugehörigen Photodioden 7 sind wiederum über ein gemeinsames teilverspiegeltes Prisma 9 in denselben Strahlengang einer Monomode-Faser 10 eingekoppelt, die ebenfalls im distalen Endabschnitt 3 mündet. Die Diagnoselaser 5 und 6 arbeiten bei dieser Ausführungsform in einem Wellenlängenbereich von 1,31 *µ*m bzw. 1,55 *µ*m, welche ein bis Größenordnung Faktor 10 unterschiedliche Absorption und damit Remission im Gewebe aufweisen. Ähnliche Unterschiede können im UV-Bereich erreicht werden (0,2 *µ*m und 0,35 *µ*m Wellenlänge). Die Laser 5,6 sind als Laserdioden ausgebildet, wobei der im 1,31 *µ*m-Bereich arbeitende Laser 5 im wesentlichen zur Topologieerfassung des zu untersuchenden Gewebebereiches dient, während der im 1,55 *µ*m-Bereich arbeitende Laser 6 zur Erfassung von Topologie und gewebespezifischen Betrachtungen dient.

Die über die Monomode-Faser 10 in den distalen Endabschnitt 3 gelangenden Laserstrahlen der Diagnoselaser 5 und 6 werden innerhalb des Endabschnittes 3 über geeignete Spiegelanordnungen zunächst einem vertikalen Scanner 12 und nachfolgend einem horizontalen Scanner 11 zugeleitet. Bei den Scannern 11, 12 handelt es sich um Silizium-Zwei-Achsen-Scanner, die aufgrund elektromagnetischer Anregung in einer vorgegebenen Frequenz um eine Achse schwingen. Die Achsen der Scanner 11 und 12 sind so angeordnet, daß die aus der Monomode-Faser 10 kommenden Lichtstrahlen in zwei um 90° zueinander versetzten Ebenen abgelenkt werden. Durch diese Ablenkung werden die punktförmigen Lichtstrahlen auf ein im wesentlichen quadratisches Feld gerichtet. Da die Schwingung der Scanner 11 und 12 durch elektromagnetische Anregung erfolgt und die Schwingungsfrequenz bekannt ist, kann eine zeitliche Zuordnung der Scanner erfolgen, so daß innerhalb der elektrischen Steuereinheit 4 zuordenbar ist, auf welchen Punkt des vorerwähnten etwa quadratischen Feldes die Lichtstrahlen momentan gerichtet sind.

Von den Scannern 11 und 12 werden die Lichtstrahlen der Diagnoselaser 5 und 6 über einen Prismenblock 13 wellenlängenabhängig jeweils einem Strahlteilerblock 14 zugeführt, um dann in einem weiteren distalwärts angeordneten Prismenblock 15 wieder vereint zu werden. Über einen distalwärts dahinterliegenden Prismenblock 16 wird dann in diesen Strahlengang der Diagnoselaser 5, der Strahlengang des Behandlungslasers 1 eingekuppelt. Dieser gemeinsame Strahlengang 17 durchläuft eine Linse 18 einer Autofokuseinrichtung, bevor er über das distalseitige Fenster 19 den distalen Endabschnitt 3 der Vorrichtung verläßt. Die Linse 18 ist über eine Magnet-Spulenanordnung, wie bei Autofokuseinrichtungen üblich, in Achsrichtung des Strahlengangs 17 bewegbar.

Das aus dem Fenster 19 austretende Licht der Diagnoselaser 5 und 6, das mittels der Scanner 11 und 12 ein Diagnosefeld überstreicht, wird von dem zu untersuchenden Material, insbesondere Gewebe, remittiert und gelangt durch das Fenster 19, die Linse 18, den Prismenblock 16 zum Prismenblock 15, wo eine wellenlängenabhängige Aufspaltung erfolgt. Über die Strahlteilerblöcke 14 wird das remittierte Licht dann wellenlängenabhängig Photodioden 20 zugeführt, die auch Teil der Autofokuseinrichtung sind. Die Autofokuseinrichtung arbeitet in Zeitfenstern, in denen die Diagnose- und Therapiefunktion unterbrochen ist, unter Ausnutzung des von den Diagnoselasern 5 und 6 am Objekt reflektierten Lichtes. Durch Verschieben der Linse 18 wird mit Hilfe der Photodioden 20 das Intensitätsmaximum des remittierten Lichtes und damit die optimal fokussierende Stellung der Linse 18 ermittelt.

Außerhalb dieses Zeitfensters gelangt das von den Diagnoselasern 5, 6 erzeugte und vom Objekt remittierte Licht durch die Strahlteilerblöcke hindurch, über den Prismenblock 13 zu den Scannern 11, 12, die es über das Prisma 9 zurück zu den Strahlteilerblöcken 8 werfen, wo es mittels der Photodioden 7 in seiner Intensität erfaßt wird. Das Signal der Photodioden 7 ist der elektrischen Auswert- und Steuereinheit 4 zugeführt, in der eine Bildpunktzuordnung entsprechend der momentanen Scannerstellung im Diagnosefeld und somit der Aufbau einer aus Bildpunkten bestehenden Matrix erfolgt, und zwar wellenlängenabhängig, zum einen für das vom Diagnoselaser 5 remittierte Licht und zum anderen für das vom Diagnoselaser 6 remittierte Licht.

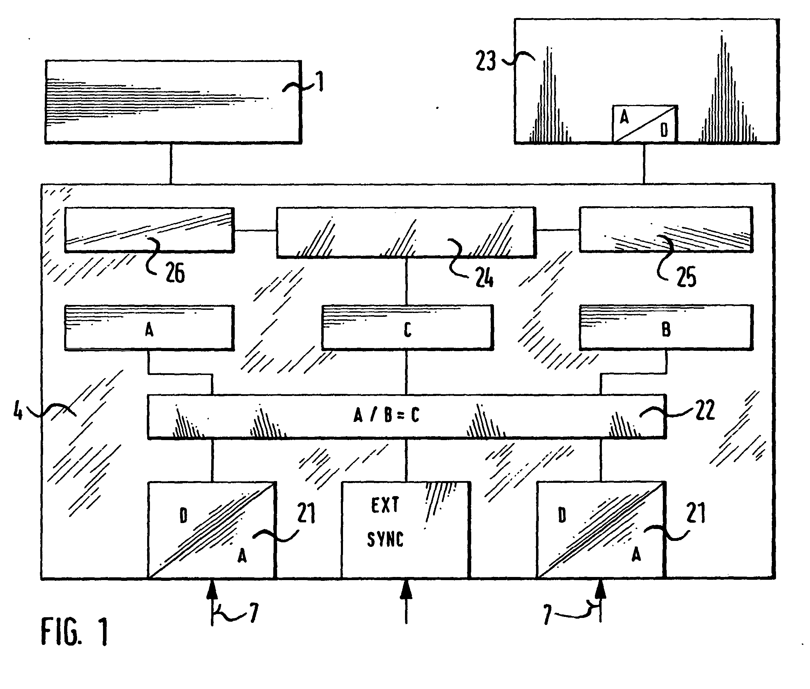

Die Signalverarbeitung innerhalb der Steuereinheit 4 ist anhand des in Figur 1 dargestellten Blockschaltbildes veranschaulicht. Das Signal der Photodioden 7 wird jeweils zunächst einem Analog-Digital-Wandler 21 zugeleitet. Das digitale Ausgangssignal dieser Analog-Digital-Wandler wiederum wird einer zentralen Bildrechnereinheit 22 zugeleitet. In dieser Bildrechnereinheit 22 werden die von den Photodioden 7 digitalisierten Daten mithilfe der Synchronisationsdaten der Scanner 11 und 12 zum einen wellenlängenabhängig zu aus einzelnen Bildpunkten aufgebauten Bildmatrizes verarbeitet, die, soweit sie vom remittierten Licht des Diagnoselasers 5 stammen, einem Bildspeicher A und, soweit sie vom remittierten Licht des Diagnoselasers 6 stammen, einem Bildspeicher B zugeführt werden.

Weiterhin werden in der zentralen Bildrechnereinheit 22 die wellenlängenabhängigen Bilddaten rechnerisch verknüpft, und zwar wahlweise durch Subtraktion oder durch Quotientenbildung. Das daraus rechnerisch ermittelte Bild wird einem Bildspeicher C zugeführt. Während die Bildspeicher A und B im wesentlichen für den Zugriff der zentralen Bildrechnereinheit zur Ermittlung der Werte für den Bildspeicher C dienen und nur wahlweise auf einem Monitor 23 darstellbar sind, wird der Bildspeicher C in regelmäßigen Abständen, beispielsweise dreimal pro Sekunde, von einer Analyseeinheit 24 abgefragt, während das durchgeschleifte Signal in einer Videoeinheit 25 aufbereitet und auf dem Monitor 23 dargestellt wird. Auf dem Monitor 23 wird also das rechnerisch verknüpfte Bild des Bildspeichers C dargestellt, das topologiebereinigt ist, also lediglich ein Bild zeigt, das insbesondere die Konturen zwischen malignem und gesundem Gewebe erkennen läßt. Durch wahlweises Einschalten der Bilder A und B kann, wenn gewünscht, auch die Topologie alleine oder zusätzlich dargestellt werden.

Innerhalb der Analyseeinheit 24 erfolgt eine elektronische Auswertung des Bildes C, insbesondere in dem zentralen Bereich, der gerade vom Behandlungslaser 1 überdeckt wird. In Abhängigkeit eines vorgegebenen, zuvor einstellbaren Schwellwertes, wird ein Signal an die Steuerung 26 des Behandlungslasers 1 abgegeben. Die Steuereinheit 4 kann so geschaltet werden, daß die Steuerung 26 bei anstehendem Signal der Analyseeinheit 24 selbsttätig den Behandlungslaser 1 einschaltet oder aber eine Freigabe einer manuellen Steuerung des Behandlungslasers 1 erfolgt, die vom behandelnden Arzt zu steuern ist. Auf diese Weise kann zuverlässig verhindert werden, daß der Behandlungslaser 1 versehentlich, zum Beispiel dann ausgelöst wird, wenn lediglich gesundes Gewebe diagnostiziert worden ist. Erst bei einem durch Einstellung des Schwellwertes vorgegebenen Mindestgehalt von malignem Gewebe im Diagnosefenster kann die Auslösung des Behandlungslasers erfolgen bzw. erfolgt diese selbsttätig.

Die Monitordarstellung erfolgt stets mit einer Wiederholfrequenz von 50 oder mehr Hz, gleichwohl die Abfragefrequenz des Bildspeichers deutlich niedriger liegen kann, beispielsweise dreimal pro Sekunde. Auf diese Weise bleibt der Bildrechnereinheit genügend Zeit, um die zeitlich versetzt kommenden Bildpunktdaten zu einem Gesamtbild zusammenzustellen und rechnerisch zu verarbeiten. Im übrigen wird die Monitordarstellung durch die Zeitfensterung für den Autofokus nicht beeinträchtigt, gleichwohl in dieser Zeit der Behandlungslaser gesperrt ist.

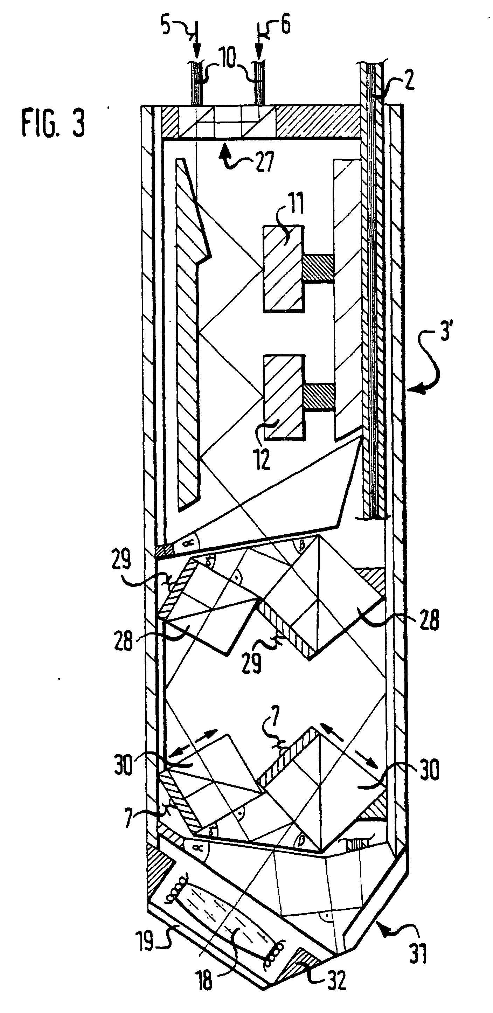

Die anhand von Figur 3 dargestellte Ausführung unterscheidet sich von der vorbeschriebenen dadurch, daß die Strahlen der Diagnoselaser 5 und 6 über gesonderte Monomode-Fasern bis zum distalen Endabschnitt 3' geführt sind, wo sie über eine geeignete Prismenanordnung 27 zusammengeführt sind. Von dort werden sie wiederum einem vertikalen und dann einem horizontalen Scanner 12 bzw. 11 zugeführt. Von dort erfolgt zunächst wieder durch entsprechende Prismenanordnung eine wellenlängenabhängige Strahlteilung. In Strahlteilerblöcken 28 wird über Photodioden 29 die Intensität des von den Diagnoselasern 5 und 6 ausgehenden Lichtes gemessen. Durch diese Erfassung der einstrahlenden Diagnoseleistung können in der Steuereinheit 4 Intensitätsschwankungen der Diagnoselaser 5 und 6 kompensiert werden, wodurch eine weitere mögliche Quelle von Fehlern ausgeschlossen wird. Die Strahlen gelangen dann über weitere Strahlteilerblöcke 30 zu einer Prismenanordnung 31, in der die Strahlen beider Diagnoselaser 5 und 6 wieder vereint und mit dem Strahlengang des Behandlungslasers 1, der ebenfalls über ein Multimode-Faserbündel 2 mit dem distalen Endabschnitt 3' verbunden ist, gekoppelt. Der gemeinsame Strahlengang von Diagnoselasern 5, 6 und Behandlungslaser 1 führt dann durch die in dieser Ausführung zur Längsachse des distalen Endabschnittes 3' schräggestellten Linse 18 zu dem ebenfalls schräggestellten distalen Fenster 19. Auch bei dieser Ausführung ist die Linse 18 Teil einer Autofokuseinrichtung, an ihrem Außenumfang mit einer Spulenanordnung versehen und in Achsrichtung verschiebbar angeordnet. Der mit der Spulenanordnung zusammenwirkende Permanent-Ringmagnet ist mit der Bezugsziffer 32 in Figur 3 gekennzeichnet.

Im Unterschied zu der Ausführung nach Figur 2 sind die Photodioden 7 zur Erfassung der Intensität des remittierten Lichtes bei der Ausführung nach Figur 3 innerhalb des distalen Endabschnittes 3' angeordnet, und zwar am Boden der Strahlteilerblöcke 30. Da hier die Intensitätsmessung vor dem Passieren der Scanner 11 und 12 erfolgt, gestaltet sich die Messung wesentlich einfacher, da zum einen die Intensität des reflektierten Lichtes noch deutlich höher ist und zum anderen durch die ganzflächige Messung eine erheblich größere Lichtmenge zur Messung zur Verfügung steht. Insofern wird diese Anordnung gegenüber der anhand von Figur 2 dargestellten zu bevorzugen sein.

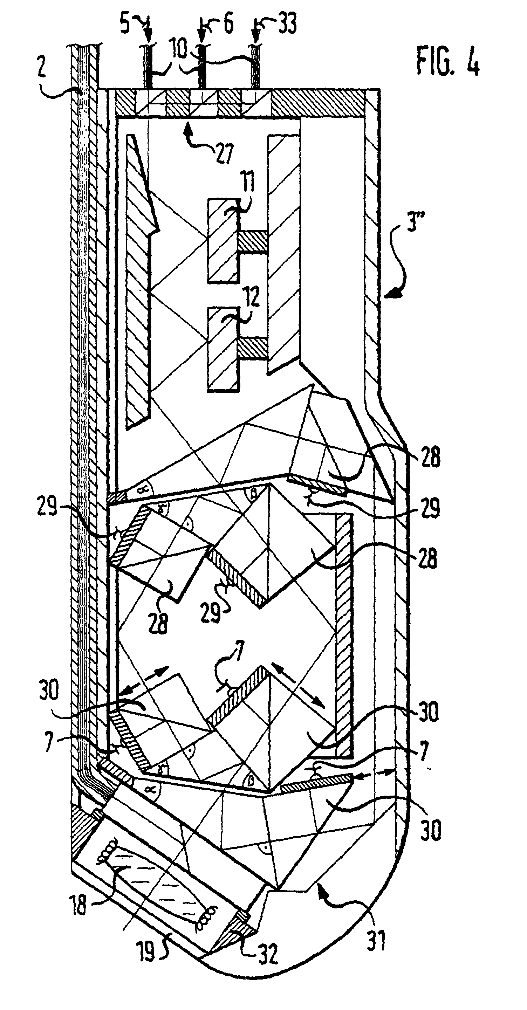

Auch bei der anhand von Figur 4 dargestellten dritten Ausführungsvariante befinden sich die Photodioden 7 innerhalb des distalen Endabschnittes 3". Der Aufbau des distalen Endabschnittes 3" entspricht im wesentlichen dem anhand von Figur 3 dargestellten, weshalb dieser hier nicht noch einmal im einzelnen beschrieben wird, er ist anhand der gleich verwendeten Bezugszeichen nachvollziehbar. Die in der Figur angegebenen Winkel α, β und γ sind dort wie folgt gewählt:
α etwa 22,5°,
β etwa 33,75°,
γ etwa 45°.

Die Winkel α und β stimmen mit denen der Ausführung nach Figur 3 überein.

Der wesentliche Unterschied zu der vorbeschriebenen Ausführung liegt darin, daß drei Diagnoselaser, 5, 6 und 33 vorgesehen sind. Die Wellenlängen dieser drei Diagnoselaser sind so gewählt, daß ein Laser im Rotbereich, ein Laser im Grünbereich und einer im Blaubereich arbeitet, so daß sich die Strahlen zu natürlichem weißen Licht addieren. Entsprechend sind die Prismenanordnungen modifiziert, und es sind drei Photodioden 29 zur Erfassung von Leistungsschwankungen der Diagnoselaser und drei Photodioden 7 zur wellenlängenabhängigen Intensitätsermittlung vorgesehen. Die Steuereinheit 4 ist entsprechend angepaßt, wobei neben einem weiteren Analog-Digital-Wandler 21 auch ein weiterer Bildspeicher vorgesehen ist, so daß durch Abfrage von drei Bildspeichern auf dem Monitor 23 ein natürliches Farbbild wahlweise zu dem rechnerisch verknüpften Bild darstellbar ist. Dies hat den besonderen Vorteil, daß der behandelnde Arzt sich unabhängig vom rechnerisch ermittelten Diagnoseergebnis auch ein natürliches Bild der zu diagnostizierenden Gewebestelle, also des von den Diagnoselasern bestrahlten Diagnosefeldes machen kann.

## Patentansprüche

1. Vorrichtung zur endoskopischen Diagnose und Behandlung von Gewebe, insbesondere von malignem Gewebe im menschlichen oder tierischen Körper, mit einem Behandlungslaser (1) und mit mindestens einem Diagnoselaser (5, 6), mit einer Empfangseinrichtung (7), mit einer Steuer- und Auswerteinrichtung (4) und mit einem endoskopischen Instrument (3), nahe dessen distalem Ende die Lichtstrahlen aller Laser austreten, **dadurch gekennzeichnet**, dass mindestens eine Scaneinrichtung (11, 12) vorgesehen ist, welche die Lichtstrahlen des Diagnoselasers (5, 6) über ein Diagnosefeld lenkt, und dass eine Fotodiode (7) als Empfangseinrichtung vorgesehen ist.

2. Vorrichtung nach Anspruch 1, **dadurch gekennzeichnet**, dass mindestens zwei Diagnoselaser (5, 6) unterschiedlicher Wellenlänge vorgesehen sind und dass jedem Diagnoselaser (5, 6) eine Fotodiode (7) zugeordnet ist.

3. Vorrichtung nach Anspruch 1 oder 2, **dadurch gekennzeichnet**, dass die Lichtführung zu den Fotodioden (7) durch Strahlteilerblöcke, Prismen und/oder Spiegel und damit unter Vermeidung des Einsatzes von Lichtwellenleitern erfolgt.

4. Vorrichtung nach einem der vorhergehenden Ansprüche, **dadurch gekennzeichnet**, dass die optischen Achsen von dem Behandlungslaser (1) und von den Diagnoselasern (5, 6) mindestens am distalen Austrittsende der Vorrichtung übereinstimmen.

5. Vorrichtung nach einem der vorhergehenden Ansprüche, **dadurch gekennzeichnet**, dass drei Diagnoselaser (5, 6, 33) unterschiedlicher Wellenlängen vorgesehen sind.

6. Vorrichtung nach einem der vorhergehenden Ansprüche, **dadurch gekennzeichnet**, dass die Diagnoselaser (5, 6, 33) jeweils in den Wellenlängenbereichen rot, grün und blau arbeiten.

7. Vorrichtung nach einem der vorhergehenden Ansprüche, **dadurch gekennzeichnet**, dass die Fotodiode / Fotodioden (7) in einem distalen Endabschnitt (3) des endoskopischen Instrumentes angeordnet sind.

8. Vorrichtung nach einem der vorhergehenden Ansprüche, **dadurch gekennzeichnet**, dass die Scaneinrichtung (11, 12) in dem distalen Endabschnitt (3) des endoskopischen Instrumentes angeordnet ist.

9. Vorrichtung nach einem der vorhergehenden Ansprüche, **dadurch gekennzeichnet**, dass die Strahlengänge der Diagnoselaser (5, 6, 33) vor dem Auftreffen auf eine horizontale und eine vertikale Scaneinrichtung (11, 12) zusammengeführt sind, wobei die Scaneinrichtungen (11, 12) im distalen Endabschnitt (3) des endoskopischen Instrumentes angeordnet sind.

10. Vorrichtung nach einem der vorhergehenden Ansprüche, **dadurch gekennzeichnet**, dass eine Fotodiode (7) selektiv das vom Bestrahlungsort reflektierte Licht im Wesentlichen einer Wellenlänge erfasst, wobei die Wellenlänge der des zugeordneten Diagnoselasers (5, 6, 33) entspricht.

11. Vorrichtung nach einem der vorhergehenden Ansprüche, **dadurch gekennzeichnet**, dass die Fotodioden (7) im distalen Endabschnitt (3) zwischen dem distalen Ende und den Scaneinrichtungen (11, 12) angeordnet sind.

12. Vorrichtung nach einem der vorhergehenden Ansprüche, **dadurch gekennzeichnet**, dass die Fotodiode (7) die Intensität des auftreffenden Lichtes erfasst, und dass in der Steuer- und Auswerteinrichtung (4) anhand des zeitlichen Signalverlaufs an der jeweiligen Fotodiode (7) und der jeweiligen Stellungen der Scaneinrichtungen (11, 12) eine bildgebende Matrix zur jeweiligen Wellenlänge ermittelt, die Matrizes unterschiedlicher Wellenlängen miteinander rechnerisch verknüpft werden und in Abhängigkeit des Ergebnisses die Freigabe und/oder Auslösung des Behandlungslasers (1) gesteuert ist.

13. Vorrichtung nach einem der vorhergehenden Ansprüche, **dadurch gekennzeichnet**, dass im distalen Endabschnitt (3) eine Autofokuseinrichtung (18, 32) angeordnet ist, deren Strahlengang mit dem der Diagnoselaser (5, 6, 33) sowie dem des Behandlungslasers (1) mindestens am distalen Ende des endoskopischen Instrumentes zusammenfällt.

14. Vorrichtung nach einem der vorhergehenden Ansprüche, **dadurch gekennzeichnet**, dass der distale Endabschnitt (3) ein Abschlussfenster (19) aufweist, dem eine Linse (18) der Autofokuseinrichtung (18, 32) vorgelagert ist, die in Richtung ihrer optischen Achse zum Zwecke der Fokussierung verlagerbar ist.

15. Vorrichtung nach einem der vorhergehenden Ansprüche, **dadurch gekennzeichnet**, dass mindestens eine Fotodiode (7) auch Teil der Autofokuseinrichtung (18, 32) bildet.

## Claims

1. A device for the endoscopic diagnosis and treatment of tissue, in particular of malignent tissue in the human or animal body, with a treatment laser (1) and with at least one diagnosis laser (5, 6), with a receiving means (7), with a control and evaluation means (4) and with an endoscopic instrument (3) close to whose distal end the light beams of all lasers exit, **characterised in that** there is provided at least one scanning means (11, 12) which guides the light beams of the diagnosis laser (5, 6) over a diagnosis field, and that there is provided a photo diode (7) as a receiving eans.

2. A device according to claim 1, **characterised in that** there are provided at least two diagnosis lasers (5,6) of a different wavelength and that to each diagnosis laser (5,6) there is allocated a photo-diode (7).

3. A device according to claim 1 or 2, **characterised in that** the light guiding to the photo-diodes (7) is effected by beam splitting blocks, prisms and/or mirrors and thus is effected avoiding the use of light-wave guides.

4. A device according to one of the preceding claims, **characterised in that** the optical axes of the treatment laser (1) and of the diagnosis lasers (5, 6) correspond at least at the distal exit end of the device.

5. A device according to one of the preceding claims, **characterised in that** there are provided three diagnosis lasers (5, 6, 33) of different wavelengths.

6. A device according to one of the preceding claims, **characterised in that** the diagnosis lasers (5, 6, 33) function in each case in the wavelength regions red, green and blue.

7. A device according to one of the preceding claims, **characterised in that** the photo diode/photo diodes are arranged in a distal end section (3) of the endoscopic instrument.

8. A device according to one of the preceding claims, **characterised in that** the scanning means (11, 12) is arranged in the distal end section (3) of the endoscopic instrument.

9. A device according to one of the preceding claims, **characterised in that** the beam paths of the diagnosis lasers (5, 6, 33) before impinging onto a horizontal and a vertical scanning means (11, 12) are led together, wherein the scanning means (11, 12) are arranged in the distal end section (3) of the endoscopic instrument.

10. A device according to one of the preceding claims, **characterised in that** a photo diode (7) selectively captures the light essentially of one wavelength, which is reflected from the irradiation location, wherein the wavelength corresponds to that of the allocated diagnosis laser (5, 6, 33).

11. A device according to one of the preceding claims, **characterised in that** the photo diodes (7) in the distal end section (3) are arranged between the distal end and the scanning means (11, 12).

12. A device according to one of the preceding claims, **characterised in that** the photo diode (7) captures the intensity of the impinging light, and that in the control and evaluation means (4) by way of the signal course with respect to time at the respective photo diode (7), and the respective positions of the scanner means (11, 12), a picture-forming matrix at the respective wavelength is evaluated, the matrices of different wavelengths are coupled numerically to one another and in dependence on the result the release and/or the actuation of the treatment laser (1) is controlled.

13. A device according to one of the preceding claims, **characterised in that** in the distal end section (3) there is arranged an automatic focussing means (18, 32) whose beam path coincides with that of the diagnosis laser (5, 6, 33) as well as that of the treatment laser (1) at least at the distal end of the endoscopic instrument.

14. A device according to one of the preceding claims, **characterised in that** the distal end section (3) comprises a closure window (19) prior to which there is mounted a lens (18) of the auto-focussing means (18,32), said lens being displaceable in the direction of its optical axis for the purpose of focussing.

15. A device according to one of the preceding claims, **characterised in that** at least one photo diode (7) also forms part of the auto-focussing means (18, 32).

## Revendications

1. Dispositif pour le diagnostic et le traitement endoscopiques de tissus, notamment de tissus malins, dans le corps humain ou animal, comprenant un laser de traitement (1) et au moins un laser de diagnostic (5, 6), un dispositif de réception (7), un dispositif de commande et de traitement de données (4), et un instrument endoscopique (3) à proximité de l'extrémité distale duquel sortent les rayons lumineux de tous les lasers, **caractérisé en ce qu**'il est prévu au moins un système de balayage ou scanner (11, 12), qui dévie et déplace les rayons lumineux du laser de diagnostic (5, 6) par-dessus un champ de diagnostic, et en ce qu'il est prévu une photodiode (7) en guise de dispositif de réception.

2. Dispositif selon la revendication 1, **caractérisé en ce que** sont prévus au moins deux lasers de diagnostic (5, 6) de longueur d'onde différente, et en ce qu'à chaque laser de diagnostic (5, 6) est associée une photodiode (7).

3. Dispositif selon la revendication 1 ou 2, **caractérisé en ce que** le guidage de la lumière jusqu'aux photodiodes (7) est effectué par des blocs de lames séparatrices, des prismes et/ou des miroirs, en évitant ainsi l'utilisation de guides d'ondes lumineuses.

4. Dispositif selon l'une des revendications précédentes, **caractérisé en ce que** les axes optiques du laser de traitement (1) et des lasers de diagnostic (5, 6) coïncident au moins à l'extrémité de sortie distale du dispositif.

5. Dispositif selon l'une des revendications précédentes, **caractérisé en ce que** sont prévus trois lasers de diagnostic (5, 6, 33) de longueurs d'ondes différentes.

6. Dispositif selon l'une des revendications précédentes, **caractérisé en ce que** les lasers de diagnostic (5, 6, 33) travaillent respectivement dans les gammes d'ondes du rouge, du vert et du bleu.

7. Dispositif selon l'une des revendications précédentes, **caractérisé en ce que** la ou les photodiode(s) (7) est ou sont disposée(s) dans un tronçon d'extrémité distale (3) de l'instrument endoscopique.

8. Dispositif selon l'une des revendications précédentes, **caractérisé en ce que** le système de balayage (scanner) (11, 12) est disposé dans le tronçon d'extrémité distale (3) de l'instrument endoscopique.

9. Dispositif selon l'une des revendications précédentes, **caractérisé en ce que** les parcours de marche des rayons des lasers de diagnostic (5, 6, 33) sont réunis avant l'impact sur un système de balayage horizontal et vertical (11, 12), les systèmes de balayage (11, 12) étant disposés dans le tronçon d'extrémité distale (3) de l'instrument endoscopique.

10. Dispositif selon l'une des revendications précédentes, **caractérisé en ce qu**'une photodiode (7) capte de manière sélective la lumière sensiblement d'une longueur d'onde, réfléchie par le lieu soumis au rayonnement, la longueur d'onde correspondant à celle du laser de diagnostic (5, 6, 33) associé.

11. Dispositif selon l'une des revendications précédentes, **caractérisé en ce que** les photodiodes (7) sont disposées dans le tronçon d'extrémité distale (3), entre l'extrémité distale et les systèmes de balayage (11, 12).

12. Dispositif selon l'une des revendications précédentes, **caractérisé en ce que** la photodiode (7) relève l'intensité de la lumière incidente, et en ce que le dispositif de commande et de traitement de données (4) détermine, à l'aide de la loi de variation du signal par rapport au temps au niveau de la photodiode respectivement considérée (7) et des positions respectives des dispositifs de balayage (11, 12), une matrice représentative d'image pour chaque longueur d'onde, combine mutuellement par calcul les matrices correspondant à des longueurs d'ondes différentes, et, en fonction du résultat, commande la validation et/ou le déclenchement du laser de traitement (1).

13. Dispositif selon l'une des revendications précédentes, **caractérisé en ce que** dans le tronçon d'extrémité distale (3) est placé un dispositif autofocus (18, 32), dont le parcours de marche des rayons coïncide avec celui des lasers de diagnostic (5, 6, 33) ainsi qu'avec celui du laser de traitement (1), au moins dans la région de l'extrémité distale de l'instrument endoscopique.

14. Dispositif selon l'une des revendications précédentes, **caractérisé en ce que** le tronçon d'extrémité distale (3) comporte une fenêtre de fermeture (19) en amont de laquelle est montée une lentille (18) du dispositif autofocus (18, 32), qui peut être déplacée dans la direction de son axe optique en vue de la mise au point.

15. Dispositif selon l'une des revendications précédentes, **caractérisé en ce qu**'au moins une photodiode (7) fait également partie du dispositif autofocus (18, 32).
